# EUROPEAN PATENT APPLICATION

(11) **EP 1 733 702 A2**
(43) Date of publication of application: **20.12.2006**
(21) Application number: 06251427.8
(22) Date of filing: 17.03.2006
(51) Int. Cl.: A61F 2/01

(54) **Integral embolic protection device and method of manufacture**

(30) Priority: 17.03.2005 US 662733 P
(71) Applicant: Nitinol Development Corporation, Fremont, California 94539 (US)
(72) Inventor: Arguello, Edward, Miramar, FL 33027 (US); DePalma, Donald, Weston, FL 33326 (US); Dwyer, Clifford J., Weston, FL 33326 (US); Mosel, Brian, Dublin, CA 94568 (US); Park, Jin S., Parsippany, NJ 07054 (US); Tanaka, Donald, Saratoga, CA 95070 (US); Wijeratne, Lalith H., Cooper City, FL 33328 (US)
(74) Representative: Belcher, Simon James

(57) **Abstract**

In accordance with the present invention, an integral embolic protection device with improved apposition to both large and small vessel walls of varying geometries cut from a single tube for enhancing the filtering of embolic material during intravascular procedures while allowing for the passage of blood is provided. The device includes a dual geometry filter basket designed to maximize apposition of the filtering portion to that of the vessel wall and capture of embolic material while not hindering blood flow which includes struts cut from a single tube that provide circumferential support to the filtering membrane and thereby minimizing or eliminating in-folding of the filter membrane while simplifying fabrication. Thin film materials may also be utilized for the filtering membrane of the filter basket. In addition one can incorporate biological and/or pharmaceutical agents in combination with the present invention.

## Description

The present invention relates to intravascular devices used to assist in medical treatment and procedures. More specifically, the present invention relates to a blood filtering system for preventing embolic material from migrating through a blood vessel during an intravascular procedure and the method of manufacture of such a device.

Atherosclerosis is a complex disease, being primarily a result of buildup in the arteries that may start as early as childhood and progress as one ages. Progression may be rapid in some people. Blood vessels may become completely occluded or more often narrowed and/or stenotic in a number of ways. A stenosis may be formed by an atheroma which is typically a harder, calcified substance which forms on the inside walls of the blood vessel. The stenosis may also be formed by a buildup of thrombus material, which may restrict blood flow through the vessel. In general, atherosclerosis is the result of any combination of fat substances, cholesterol, waste products, calcium as well as other substances being deposited on the inside lining of the artery. This buildup is often called plaque. Atherosclerosis can often lead to coronary heart disease, a major health concern today for both males and females in the United States as well as abroad.

Atherosclerosis may also lead to strokes, and other disorders because of the occurrence of blood clots, which may form in the narrowed arteries. Although plaques can grow large enough to significantly reduce the blood flow through an artery, most of the damage may occur when these plaques become fragile and rupture. These are often referred to as vulnerable plaques. When vulnerable plaques rupture they typically cause blood clots to form that may then subsequently block blood flow or break off and travel through the blood vessel to another part of the body. If either situation happens, the result may be a blocked blood vessel that supports and nourishes the heart, which may cause a heart attack. If a blood vessel that delivers blood to the brain is blocked, it may cause a stroke. If the blood supply to the legs is compromised, it may result in limb ischemia and in difficulty with walking and/or leg pain referred to as claudication, and may eventually cause gangrene.

The narrowing of an artery, also known as a stenotic lesion in the vasculature, has motivated medical professionals to develop a number of intravascular procedures which have evolved over time to treat this condition, percutaneous balloon angioplasty being the most common. Percutaneous balloon angioplasty is a procedure wherein a balloon catheter is inserted within the vasculature, and the balloon is expanded at the location of the lesion essentially compressing the stenotic buildup against the inside of the vessel wall. More recently this procedure has been augmented by the deployment of a stent or stents, at the location of the lesion subsequent to, or concurrently with the angioplasty. The stent acts as an internal scaffold within the vessel, retaining an open lumen and preventing further re-narrowing of the vessel. Generally, stents are primarily of two types, balloon expanding and self-expanding. As the terms indicate, balloon-expanding stents are deployed/expanded *in-vivo* with the assistance of a balloon, while self-expanding stents may utilize shape-memory materials such as nitinol. The use of such alloys as nitinol (Nickel-Titanium alloy), having shape memory characteristics, suitable biocompatibility, and designed to be inserted into one's vasculature, is known in the art. These so-called shape memory alloys have the ability to return to their original shape when exposed to the correct temperature conditions. The shape memory characteristics of these alloys, allow the devices to be deformed to facilitate their insertion into a body lumen or cavity. Upon being exposed to higher temperatures within the body results in the device returning to its original programmed shape. Thus one can employ the shape memory property to expand the device to its original shape after being delivered through the vasculature in a reduced profile or compressed state. Nitinol can also have super-elastic characteristics, which allow the device fabricated from such a material as nitinol, to be deformed and restrained in the deformed condition in order to facilitate the insertion into a patient's vasculature. This deformation causes a phase transformation of the material. Once the device with super-elastic characteristics is delivered, the restraint on the super-elastic material can be removed thus reducing the stress and allowing the previously compressed super-elastic member to return to its original pre-programmed and un-deformed shape, which results in a transformation back to the original phase.

While widespread intravascular procedures, particularly angioplasty procedures have been extremely successful; the procedure itself may result in development of an embolus. For example, during stent deployment and positioning, abrasion of the vessel wall may dislodge material resulting in an embolus. An embolus circulating within the blood vessels may lead to occlusion of a vessel and/or formation of clots within the vasculature and/or body organs. Although the occurrence of this can be minimized with careful, meticulous and proper technique, when such an event does happen it may have serious consequences to the patient. By adequately capturing and/or filtering the material traveling in the blood responsible for causing such an event one can avoid the serious consequences that may result without such safeguards.

Multiple approaches to address capturing and/or filtering of the embolic debris/material from blood have been attempted. These include baskets, nets, suction, and even chemical modification of the debris (see US-5,053,008, which utilizes and requires an additional conduit to transport lysing agents to the trapped embolus). Use of vascular filters in the Vena Cava for capturing emboli has been disclosed (see US-4,727,873 and US-4,688,553). The designs of the Vena Cava filters continue to improve addressing such issues as fit and preventing migration (see US-6,443,972). Distal Protection devices using filtering baskets although similar to Vena Cava filters in function are typically temporarily positioned within the lumen, whereas Vena Cava filters are typically implanted within the vessel, most often the inferior Vena Cava, as the name implies. The use of a filtering basket for distal protection positioned downstream from the procedure to capture the debris/material during an intravascular procedure is one such method. Because these basket type devices must be introduced into the vasculature and travel within the vasculature to be ultimately positioned to a location somewhat distal or downstream to the region of interest, most, if not all, incorporate and utilize concepts and features that make the essential delivery through the vessel less traumatic (see US-6,391,044). This can be accomplished by using a reduced size or compressed version of the apparatus. This allows one to deploy the apparatus to its normal working size when the apparatus is at the location of interest within the vessel. Because of the variability of vessel diameters, sizing of the device is important to ensure adequate expansion of the device once positioned in the target vessel. Undersized devices when used in larger vessels may not be able to extend the filtering area of the device to the outer margins of the vessel while oversized devices utilized in smaller vessels may have multiple folds in the flow path hindering blood flow. Both of these situations are undesirable and although properly sized devices to the corresponding vessel diameter may adequately accommodate proper filtering without hindering blood flow, it would be beneficial for a single device to accommodate multiple vessel diameters without hindering blood flow.

The majority of these basket-type devices employ "expandable filters," meaning they may be fabricated from shape-memory materials which have the property that when exposed to the relatively elevated temperature within the body, they return to their initial programmed size. Alternately, on can rely on the superelastic property by removing the restraint on the geometry. These devices are generally placed distal, or downstream of the stenosis in order to capture any fragments, debris, and/or embolic material, that may be dislodged or occur as a result of the presence and use of the device during an intravascular procedure. The downstream placement of the device takes advantage of the blood flow within the vasculature, which will transport the undesirable material with it. The filtering membrane of the device is typically designed so as to allow blood flow through the membrane while limiting passage of the larger sized fragments and debris such as micro and macro emboli. These fragments, debris, and/or embolic material could potentially be carried beyond the device location with the blood flow downstream if not for such a filtering device as described herein. While this method performs fairly well capturing a substantial portion of the items intended to be captured; many of these designs are optimized for circular vessels. While outer vessel shapes are circular or slightly elliptical, the internal geometry of a vessel may often be non-circular as in an oval or elliptical shape or may even take the form of other non-circular geometries. Specifically, when calcification is present within the vessel, the internal vessel geometry is often irregular. Furthermore, vessel cross-sectional shape may vary with the type and location of vessel and vary across patients as well. Moreover, due to the circulation of blood through the vessel and resulting forces, a dynamic situation exists, which may produce additional geometry changes to the normal vessel shape. Thus expandable filters, which are generally designed for circular vessels, may result in a lack of apposition against the vessel wall over at least some portion of the internal circumference of the vessel wall when one takes into account the additional factors described above. Such a gap or leak path may occur when the resulting geometry of the expanded device is circular while the inner luminal cross-sectional shape of the vessel is more often non-circular. Such resulting gaps, between the device and inner luminal surface, may allow the emboli that the device is designed to capture, adequate room to pass through such a gap between the inner wall of the vessel and the outer confines of the device. When this occurs, the primary purpose of the device, which in this case is to capture fragments, debris and/or embolic material, is defeated, because the unfiltered flow path will allow for passage of the emboli. Even when the vessel itself is circular, conformance of a circular filter to the internal lumen of the vessel may not be optimal if "in-folding" is present. "In-folding" is the situation when the unsupported membrane of the system folds in at positions located between the strut locations where the membrane is supported by the struts. This situation can produce gaps between the inner vessel wall and the membrane even in the idealized circular vessel at locations between adjacent struts. In-folding may also occur when an oversized device (one that is sized larger then the vessel it will be placed in) is utilized in order to ensure adequate vessel coverage. In this situation, when the struts of the oversized device make contact with the vessel wall, the device ceases to expand. As a result, this limited expansion is short of its fully expanded programmed size and as such the membrane is not fully taught. Thus the remaining slack present in the membrane may lead to in-folding and thus allow for an unfiltered flow path. Moreover, even in the absence of in-folding, utilization of a circular-type device in a truly circular vessel may not adequately conform to the internal lumen upon vessel loading and/or deformation because the resulting device deformation may not adequately match the deformation of the vessel. This dissimilar deformation may thereby result in gaps or leak paths between the inner vessel wall and the device upon loading and/or deformation of the vessel.

While circumferential apposition of the vessel is important, the axial dimensions and axial design considerations of distal protection devices are also important. Most distal protection devices are designed so that placement of the device is distal to the lesion in order to capture the dislodged fragments and/or embolic debris that may be produced as a result of the procedure itself and flow downstream. Since lesion length is variable and may be of any length, devices that are longer in length than the remaining non-diseased operative vessel length distal to the lesion may present a problem for the interventionalist using a device which requires a length longer than the length of the non-diseased vessel distal to the lesion. The remaining non-diseased operative vessel length distal to the lesion may be shorter than the overall device length due to a vessel bifurcation, or a significant change in vessel direction, or reduction in vessel diameter. When the non-diseased operative vessel length is shorter than the overall device length it may result in a distal protection device not being able to accommodate these vessel configurations.

The overall size of these devices is quite small, with the compressed diameter of these devices approximately 0.040" to facilitate delivery through the vasculature. This presents unique manufacturing challenges. Many of the existing type of filtering devices are fabricated from wire strands that are assembled together to form an integral unit. Due to the small size of these products and the number of assembly steps to form the product, achieving consistency of final product at reasonable costs can be challenging. In most devices that are fabricated from wire strands, the diameter of the individual wires used is on the order of two to five thousandths of an inch (0.002" to 0.005") (0.051mm to 0.127mm), which is approximately equivalent to the diameter of a human hair. Consistent assembly of such small diameter strands can be difficult, extremely complicated, and as such expensive to manufacture.

As these devices are designed to capture embolic material while allowing for the flow of blood to pass unhindered, those devices that are successful in capturing the embolic debris may result in impeding blood flow as the load of embolic material increases. This situation may arise when the embolic load on the filter results in the perfusion holes of the filter being partially or totally occluded. Occlusion of these perfusion holes may result in the allowable blood flow downstream of the device being compromised or significantly reduced, which may cause a harmful situation to the tissues downstream of the device.

Accordingly, there is a need for a embolic protection device with improved filtering and vessel apposition that can allow for capturing of embolic debris regardless of vessel size or shape or various loading regimes encountered by the vessel and a need for a embolic protection device which is designed for ease of manufacturability. Furthermore there is a need for a device that can handle an embolic load with out compromising downstream blood flow. A need exists for a device that achieves the above while also minimizing the overall length of such a device and which allows one to accommodate various vessel diameters without the need for additional sizes thereby reducing complication rates and improving patient outcome.

The integral embolic protection device and method of manufacture in accordance with the present invention overcomes the disadvantages and shortcomings of currently available devices and satisfies the unmet needs of maximizing capture of embolic debris by improved vessel apposition with a design that simplifies manufacturing and fabrication of such a device. Moreover, the method of manufacture of this device reduces assembly and results in manufacturing cost improvements by utilizing laser cutting techniques acting on nitinol tubes, nitinol thin films and polymeric membranes which may enhance filtering capacity without compromising downstream blood flow while additionally minimizing overall length of the device in order to maximize placement and usage options of the device for the interventionalist in difficult cases.

The present invention relates to an apparatus for intravascular filtering, capable of capturing emboli in blood flowing within the vasculature and a method of manufacturing the device. The filtering device may be fabricated from a single tube and comprises an filtering membrane attached to a skeleton frame which is configured to deploy radially outward which may be relative to a centrally located guide wire. Expansion of the filtering device with improved vessel conformance is accomplished in a fashion resulting in improved filtering of blood in both circular and non-circular vessels and for vessels both large and small as well as when the vessel itself encounters various internal and/or external loading regimes. Additionally, by cutting the geometry from a single tube, manufacturing process steps and costs are significantly reduced. Furthermore the incorporation or application of biological and/or pharmaceutical agents can provide additional benefits when used in combination with the present invention.

In an exemplary embodiment of the present invention, the distal supporting collar and at least one primary strut may be cut from a single nitinol tube thereby eliminating the need for assembly of the supporting strut or struts to the collar. Alternately, at least one primary strut and the proximal supporting collar may be cut from a single tube.

In yet another exemplary embodiment of the present invention, in addition to at least one supporting collar and at least one primary supporting strut being cut from the single nitinol tube, an additional supporting collar spaced a distance away from the existing supporting collar with the supporting skeleton inter-disposed between the two collars may be cut from the same tube, resulting in all three aspects of the device integrally attached and ready for additional finishing and shaping steps.

In a third exemplary embodiment of the present invention, improved conformance in both circular and non-circular vessels is achieved and in-folding of the filtering aspect is addressed. In this exemplary embodiment of the present invention the struts are cut from the tube to allow for the path of a supporting strut to be both axial and circumferential. The circumferential portion of the cut strut provides support to the membrane resulting in improved apposition of the membrane against the inner vessel wall along the circumference of the device. Additionally, by using laser-cutting techniques on the filtering membrane to create longitudinal slits one can provide a membrane that can accommodate multiple vessel sizes. When utilized in vessels of larger diameter the laser cut slits in addition to or in lieu of holes, allows the membrane to expand facilitating reaching the outer margins of the vessel lumen internal diameter while still filtering the blood so as to capture embolic debris. When utilized in vessels of smaller diameter, the laser cut slits in addition to or in lieu of holes, prevents the filtering membrane from folding into the lumen, which would in certain instances impede blood flow through the filtering membrane. Thus incorporation of the laser cut slits either in addition to, or in lieu of holes, allows one to better accommodate a range of vessel diameters with a single device.

In a fourth exemplary embodiment of the present invention, filtering capacity is maximized while concurrently not compromising downstream blood flow. This is achieved with a unique dual geometry of the filtering membrane of the device that both allows for the embolic material to be directed toward the central portion of the device as well as provide for a repository of the embolic material without compromising blood flow through the filtering membrane. The repository is preferably a substantially cylindrical portion of the device located distal to a filtering region which occupies a larger coverage area than the substantially cylindrical portion in the expanded state. This filtering region, which is larger than the substantially cylindrical portion, may be either substantially conical or substantially spherical or simply larger in area than the filtering region in the central substantially cylindrical portion. Both the substantially conical or spherical portions and the substantially cylindrical portion of the device have a filtering membrane that allows for the passage of blood flow through said membrane. This is accomplished with laser-drilled holes or slits cut into the membrane. These holes and/or slits may be located in variety of patterns in order to maximize flow while maintaining filtering capacity. The substantially conical portion or substantially spherical portion results in the embolic material being directed towards the central portion of the flow path where it is then captured and resides in the substantially cylindrical portion of the device. This is achieved without introducing excessive turbulence in the blood flow path, which may be problematic. By directing the embolic material toward the central substantially cylindrical portion, the outer substantially conical or substantially spherical portion remains free of embolic material and thus continues to allow for an adequate level of blood to flow through the device since there is no embolic material blocking the blood's passage through the filtering membrane in the substantially conical or substantially spherical portion.

Additionally, the filtering membrane can accommodate additional embolic debris by shaping the membrane at the distal portion to include one or more "lobes" that allow for an increased volume to accommodate embolic debris. These "lobes" are formed into the membrane and are in addition to the dual geometry resulting from the struts or skeleton frame cut from the tube for which the membrane is attached. The substantially cylindrical portion of the device may incorporate shaped capture lobes in the distal portion of the substantially cylindrical portion of the membrane, which may allow for increased embolic load to be accommodated. The distal shaped lobes are created when the membrane is formed. One such method of forming the membrane utilizes a mandrel of the desired shape, which is then dipped into the polymeric solution. Upon curing, the polymeric solution forms a membrane over the mandrel mimicking the shape of the mandrel. Upon removal of the mandrel; the membrane is left with the desired shape.

Polyurethanes are one such class of polymers that may be fabricated into a membrane in this fashion. Alternately, the use of thin-film nitinol may be employed to use in place of the polymeric membrane and serve as the filtering membrane both with and without the distal lobes. The use of thin film nitinol has the added advantage of shape memory and/or superelastic properties that may be utilized for creating "tuned" or "programmed" shapes as well as to assist with the expansion of the device. An additional benefit with the use of thin film is that thin film would provide additional structure to the distal portion of the device thereby eliminating the need for a distal supporting skeleton and a distal support collar.

The incorporation or application of biologically active or pharmaceutically active compounds with the present invention is a further object of this invention and is an improvement to methods and/or devices which require the use of a conduit to deliver the agent to the desired location. Compounds such as those identified below may be applied as coatings on these devices and may be used to deliver therapeutic and pharmaceutical agents which may include: anti-proliferative/antimitotic agents including natural products such as vinca alkaloids (i.e. vinblastine, vincristine, and vinorelbine), paclitaxel, epidipodophyllotoxins (i.e. etoposide, teniposide), antibiotics (dactinomycin (actinomycin D) daunorubicin, doxorubicin and idarubicin), anthracyclines, mitoxantrone, bleomycins, plicamycin (mithramycin) and mitomycin, enzymes (L-asparaginase which systemically metabolizes L-asparagine and deprives cells which do not have the capacity to synthesize their own asparagine); antiplatelet agents such as G(GP) ll_{b}/lllₐ inhibitors and vitronectin receptor antagonists; anti-proliferative/antimitotic alkylating agents such as nitrogen mustards (mechlorethamine, cyclophosphamide and analogs, melphalan, chlorambucil), ethylenimines and methylmelamines (hexamethylmelamine and thiotepa), alkyl sulfonatesbusulfan, nirtosoureas (carmustine (BCNU) and analogs, streptozocin), trazenes - dacarbazinine (DTIC); anti-proliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine {cladribine}); platinum coordination complexes (cisplatin, carboplatin), procarbazine, hydroxyurea, mitotane, aminoglutethimide; hormones (i.e. estrogen); anti-coagulants (heparin, synthetic heparin salts and other inhibitors of thrombin); fibrinolytic agents (such as tissue plasminogen activator, streptokinase and urokinase), aspirin, dipyridamole, ticlopidine, clopidogrel, abciximab; antimigratory; antisecretory (breveldin); anti-inflammatory: such as adrenocortical steroids (cortisol, cortisone, fludrocortisone, prednisone, prednisolone, 6α-methylprednisolone, triamcinolone, betamethasone, and dexamethasone), non-steroidal agents (salicylic acid derivatives i.e. aspirin; para-aminophenol derivatives i.e. acetaminophen; indole and indene acetic acids (indomethacin, sulindac, and etodalac), heteroaryl acetic acids (tolmetin, diclofenac, and ketorolac), arylpropionic acids (ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate); immunosuppressives: (cyclosporine, tacrolimus (FK-506), sirolimus (rapamycin), azathioprine, mycophenolate mofetil); angiogenic agents: vascular endothelial growth factor (VEGF), fibroblast growth factor (FGF); angiotensin receptor blockers; nitric oxide donors; antisense oligionucleotides and combinations thereof; cell cycle inhibitors, mTOR inhibitors, and growth factor receptor signal transduction kinase inhibitors; retenoids; cyclin/CDK inhibitors; HMG co-enzyme reductase inhibitors (statins); and protease inhibitors.

The use of compounds in conjunction with the present invention can provide distinct clinical advantages over existing therapies and/or devices. More specifically, compounds that are capable of causing lysis or degradation of the embolic debris can be incorporated into the filtering portion of the present invention, specifically the substantially cylindrical filtering portion where the embolic debris is directed. A factor to consider in the selection of such a compound is the origin of the debris be it thrombus, plaque, atheroma, or any other form representing an embolus. As the mesh and or pore size of the filtering aspect of the present invention decreases, more embolic material may become trapped in the filtering mechanism of the present invention, thereby increasing the load on the filtering portion. While small emboli (typically smaller than 100 microns) are not a major concern because of the body's natural ability to enzymatically degrade, digest or lyse the emboli, the embolic load on the filter itself can be overloaded and result in formation of a thrombus if the blood flow is significantly slowed to the point which allows for a thrombus formation. In this situation the incorporation or application of compounds, which can degrade trapped emboli, can be beneficial and when used in conjunction with the dual geometry filtering shape can ensure that adequate blood flow will reach tissues located distal of the device. Some exemplary suitable compounds may include: Tissue Plasminogen(TPA); Streptokinase(SK); Reteplase; Tenecteplase; Urokinase; Lanoteplase; Staphylokinase; and/or Nadroparin(anti-factor Xa). In addition, the dual geometry-filtering portion of the present invention may incorporate an antithrombotic and/or antithrombogenic agent to prevent the formation of a thrombus. Some exemplary compounds may include: Heparin; Fragmin (dalteparin, low MW Heparin); a monoclonal antibody such as that sold under the trade name ReoPro (abciximab, antiplatelet antibodies) Acenocoumarol; Anisindione; Dicumarol; Warfarin; Enoxaparin (Lovenox); Anagrelide (Agrylin); Indomethacin (Indocin); Dipyridamole; Clopidogrel; Aggrenox; and/or Coumadin. Furthermore, an affinity-binding compound may also be incorporated with the filtering aspect of the present invention by itself or in combination with other compounds. Affinity-binding compounds can promote the binding and/or adhesion of embolic material thus facilitating entrapment of embolic material and subsequent removal from the blood stream. Compounds such as these would preferably be located in the substantially cylindrical portion of the device. Whether incorporated into the supporting strut or filtering membrane by methods such as chemical surface treatments, bombardment, placement into reservoirs, or in the case of polymeric struts and membranes, blended with the material itself, or by application of a coating to the struts and/or membranes with a compound, any identified compound or combination of identified compounds may be used. Furthermore any number of compounds may suggest themselves to one who is skilled in the art and may be utilized in connection with the present invention alone or in combination with other compounds.

Whereas fabrication and assembly of embolic protection devices constructed of wire strands can involve numerous labor-intensive process steps, designs in accordance with the present invention simplify manufacturing. In accordance with the present invention, a laser may be programmed to cut the desired shape from a hollow tube be it polymeric or metal, preferably a nitinol tube; after cutting, a deburring operation to remove burrs as well as the cutout portion of the tubular structure may be followed by a heat treatment to "program or tune" the desired shape of the device; the heat treating and/or annealing of the device may employ the use of a mandrel to ensure the desired shape is achieved. The device in accordance with the present invention may undergo finishing steps such as electro-polishing and/or acid etching. In accordance with the present invention, the filtering membrane may be attached to the tubular structure by immersion into a liquid urethane bath; the membrane can then be perforated by undergoing laser drilling to produce approximately one hundred micron sized holes. Thin film technology may also be employed to produce the filtering membrane. The supporting struts cut from a unitary tube would provide the supporting skeleton or frame for the membrane and as a minimum support the membrane at the proximal opening to ensure the filtering membrane upon expansion of the device achieves satisfactory apposition with the wall surface of the internal lumen. Although not required, the frame may also provide support and structure to the distal portion of the membrane as well. Although many of these similar process steps disclosed are also employed with assembled wire strand devices, it is important to note that the labor intensive assembly steps associated with wire strand devices are eliminated having substituted the programmed laser cutting of the device into a tubular structure in accordance with the present invention. Moreover, cutting patterns may be optimized to achieve certain shapes and expansion diameters such as grouping of holes, gradients of pore sizes, slits or holes alone or in combination.

In an additional embodiment of the present invention, the laser cut frame can be tuned to a multitude of shapes. One such shape incorporates a twist in the proximal portion of the supporting struts making up the skeleton frame. By incorporating a synchronous twist in each of the proximal support members one can effectively reduce the overall length of the device without affecting the expanded programmed diameter. The synchronous twist is achieved by having the proximal portion of each of the supporting skeleton frame members follow the same path as they extend from the proximal support collar which results in a reduction of the overall length of the device. This minimization of the overall length of the device allows the device to be used in the more difficult cases where the operative length of un-diseased vessel beyond the lesion may be limited. Other methods of minimizing the overall length of the device can be achieved with an "S" shaped bend in the distal portion of the strut all within a radial plane of the device such that upon expansion the additional volume is accommodated without extending the overall length of the device in the compressed or expanded condition.

Alternately, if one employs an asynchronous twist, that is to say in a first group of supporting members, the proximal portion of every other member of the skeleton frame follows the same path as they extend from the proximal support collar to the distal supporting collar, while a second group of the remaining members inter-disposed between each of the members of the first group and each following a similar path extending from the proximal support collar but a path that is different from the path the first group of members followed. Preferably in this embodiment, the paths would be mirror images of each other; in addition one may achieve other non-circular shapes that may be capable of increased expansion.

The foregoing exemplary embodiments of the present invention each provide a reliable, easy to manufacture, and simple to use device that significantly improves wall apposition of the outer confines of the device to the internal surface of the vessel wall regardless of vessel size or shape or loading regime encountered while concurrently significantly expands design flexibility and configurations achievable. Furthermore, a relatively reduced profile of the delivered device is achievable in accordance with the present invention. Moreover, any combination of the materials identified that are capable of expansion and provide for the ability to independently conform to both circular and non-circular geometries upon expansion may be utilized. As noted above, the incorporation of biological and/or pharmaceutically active agents with the present invention can be utilized for the additional purposes of preventing thrombus formation, promotion of binding, and degradation of thrombus, all of which provide a patient benefit.

Embodiments of the invention will now be described by way of example, with reference to the accompanying drawings, in which:
Figure 1A is a partial side view of the filtering device showing the presence of wire strands attached to both proximal and distal supports positioned on a guide wire.
Figure 1B shows a three-dimensional view of a filter device fabricated from wire strands with a urethane membrane attached.
Figures 2A through 2D are planar views showing examples of some flat cutting patterns for tubing which when cut on hollow tubes in accordance with the present invention will result in the cut tube upon expansion being formed into a diversity of shapes which not only provide distributed support for the membrane but allow for sufficient expansion of the tube to ensure that the extent of filtration includes the outer margins of the internal diameter of the vessel.
Figures 3A and 3B show a three-dimensional view of the device in an expanded configuration in accordance with the present invention with a polymeric filtering membrane in the dual geometry configuration.
Figure 4 is a three-dimensional view of the skeleton frame fabricated from a single tube in an expanded configuration before attachment of a filtering membrane attached in accordance with the present invention.
Figures 5A and 5B show a three-dimensional view of the device in an expanded configuration without and with the filtering membrane attached fabricated using the cutting pattern shown in Figure 2A in accordance with the present invention.
Figures 6A and 6B are three-dimensional views of the filtering device fabricating using the cutting pattern shown in Figure 2A with the membrane attached in accordance with the present invention in the single and dual geometry configurations respectively.
Figure 7 shows a three-dimensional view of the filtering device in an expanded configuration fabricating using the cutting pattern shown in Figure 2B with the dual geometry filtering membrane attached in accordance with the present invention.
Figure 8A through 8C depict the process steps of providing a tube, cutting the tube in a distinct pattern, and expanding/programming the tube to the desired shape by showing the starting, intermediate, and end state of the tube before cutting, during cutting, and after shaping in accordance with the present invention.
Figure 9 shows a three-dimensional view of the expanded filtering device with the membrane attached and the presence of the laser cut holes in the membrane in accordance with the present invention.
Figure 10 schematically shows a partial side view of the expanded filtering device with the substantially conical filtering membrane and struts having an intermediate circumferential portion in accordance with the present invention.
Figure 11 A schematically shows a partial side view of the expanded filtering device with dual geometry having a substantially conical and substantially cylindrical filtering membrane in accordance with the present invention.
Figure 11B schematically shows a partial side view of the expanded filtering device with dual geometry having a substantially spherical and substantially cylindrical filtering membrane in accordance with the present invention.
Figures 12A & 12B show three-dimensional side and front views of the dual geometry device with the synchronous twist present in the proximal portion of the supporting skeleton in accordance with the present invention.
Figures 13A & 13B are planar sectional views of both a device without support to the circumferential aspect resulting in the presence of in-folding, and of a device with the circumferential aspect of the filtering membrane supported by the strut portion of the laser cut tube resulting in improved vessel conformance in accordance with the present invention and where in-folding is minimized and/or completely eliminated because of the substantially circumferential 360 degree sealing of the filter membrane against the vessel wall.
Figures 14A and 14B show an isometric and side view of an expanded filtering device with a membrane attached in accordance with the present invention.
Figure 15 shows a side view of the device having proximal supporting struts which loop back to the proximal support tube all of which are cut from the same tube in accordance with the present invention.
Figure 16A and 16B represent a schematic of a cross sectional view through the supporting skeleton wherein figure 16A represents a typical wire strand device (prior art) and figure 16B represents the cross section of the structural elements of the skeleton frame when cut from a tube.
Figure 17A and 17B represent a schematic showing the reduction of overall length when an "s-shaped" bend is incorporated into the strut.

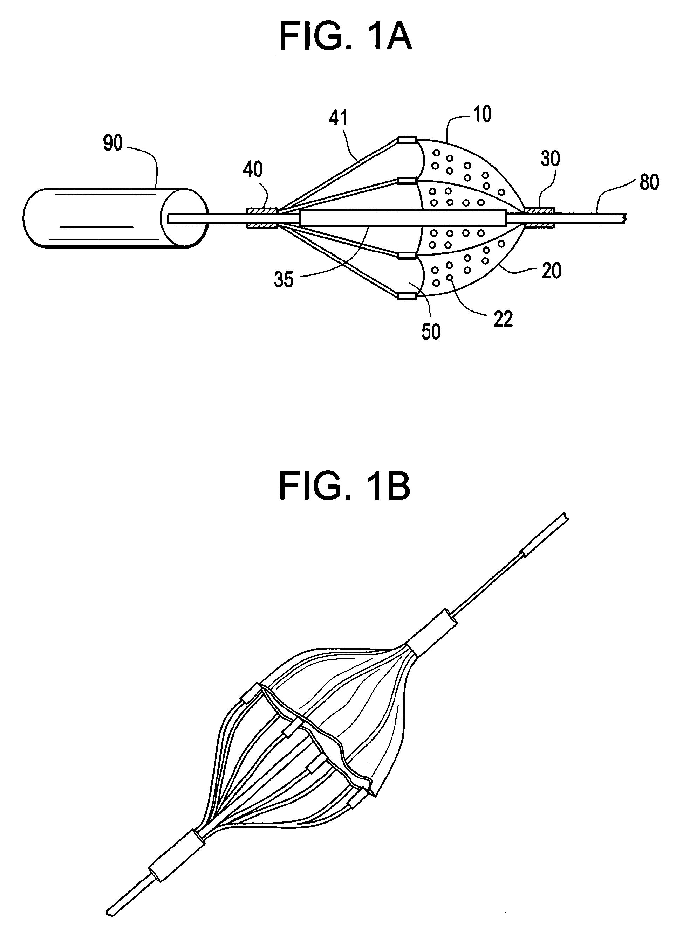

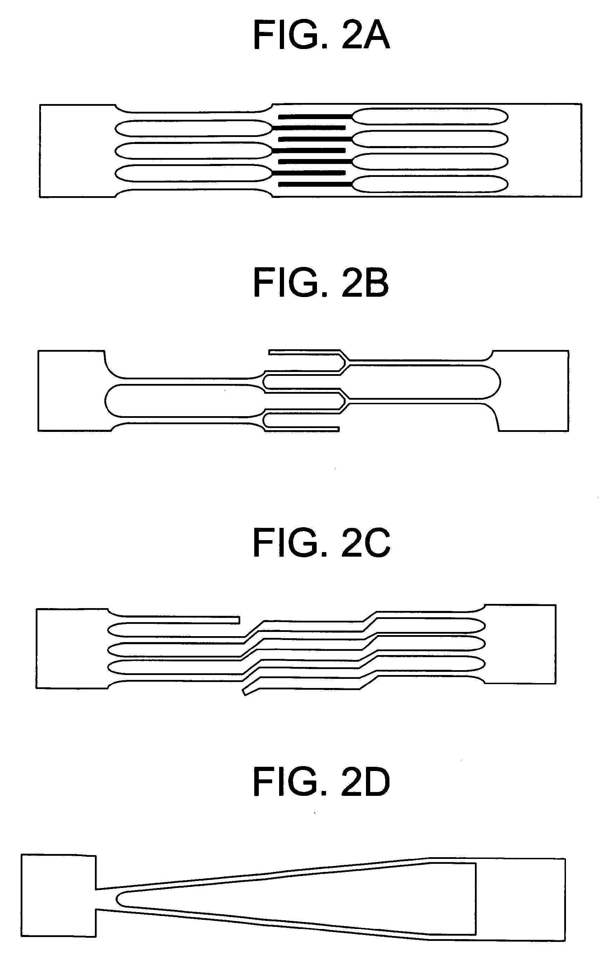

Figure 1A shows a side view of a filtering device positioned on a guide wire (80). In the assembled wire strand devices, distal struts (20) and tension members (41) are assembled and attached to the distal supporting collar (30) and the proximal supporting collar (40). In accordance with the present invention, supporting collars (30 & 40) represent the distal and proximal ends of the same tube while supporting struts (20) and tension members (41) are simply what remains from the tube being the portion not removed by laser cutting and thus are formed from the single tube. Figure 1B shows this configuration in an actual product with the membrane (50) attached. Furthermore, filter membrane (50) can be optimized for filtering capacity by incorporating a combination of pores with consistent or varying sizes and distributions. In the image shown, the filtering pores (22) are of one size, however both diameter, placement, and even shape as for example slits, can be used to maximize filtering capacity while maintaining adequate flow through the filter. In figures 2A through 2D, planar views of exemplary two-dimensional cutting patterns are shown which when laser cut on a three dimensional tube will yield different three dimensional shapes, all of which are suitable for an embolic protection device in accordance with the present invention. These cutting patterns result in skeletons of various configurations being formed which ultimately may provide the support for the filtering membrane (50), which is subsequently attached.

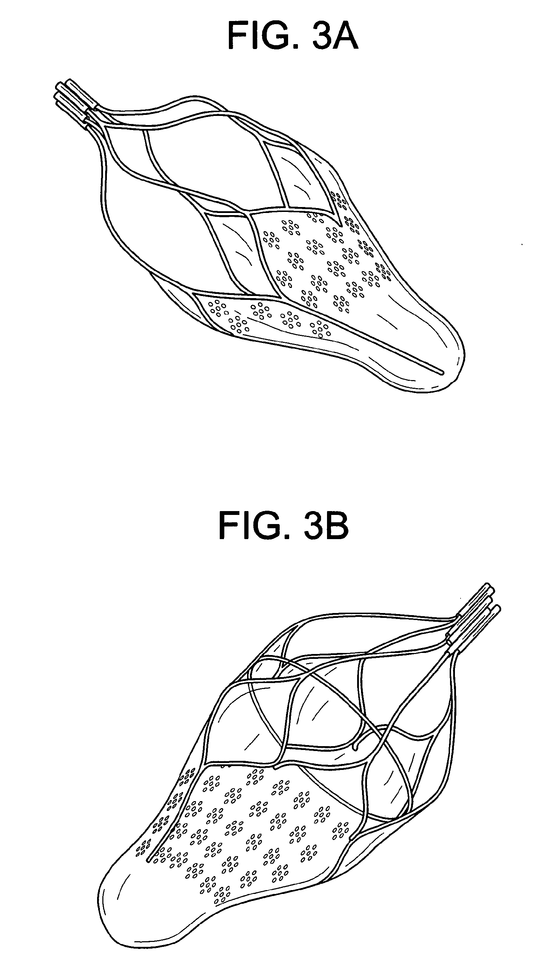

In figures 3A and 3B, a three dimensional views of the expanded configuration of the filtering device with the dual geometry filtering membrane attached is shown in accordance with the present invention. In this view one can see both the substantially conical filtering portion of the membrane as well as the substantially cylindrical portion of the membrane located distal to the conical portion, both portions may have laser cut holes and/or slits to aid in the filtration and fitting of the expanded configuration. The laser-drilled holes through the membrane can be seen in this view. As previously stated varying hole sizes can be drilled through the membrane, as can different patterns of holes in an effort to optimize the filtering effectiveness. A combination of pores with consistent or varying sizes and distributions may also be utilized. Use of laser cut slits may allow for increased flexibility and adaptability of the device to multiple vessel diameters by preventing folding in of the device when used in undersized vessels while also allowing for increased expansion of the filtering membrane when used in oversized vessels. These views also show the synchronous twist aspect of the proximal skeleton effectively reducing the overall length of the device to allow for use in the more difficult cases where the non-diseased operative length of vessel may be limited.

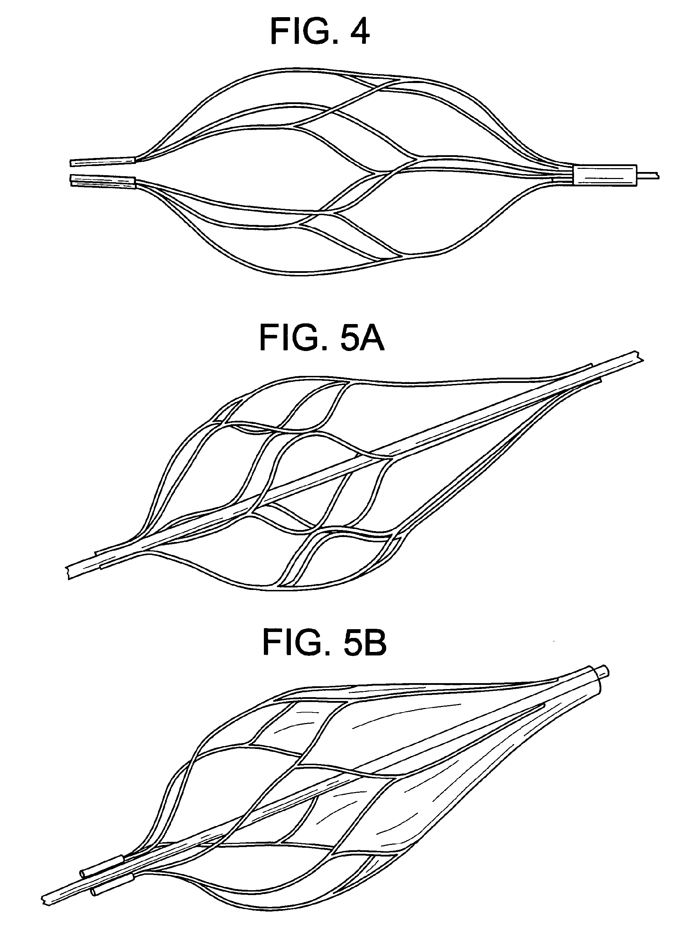

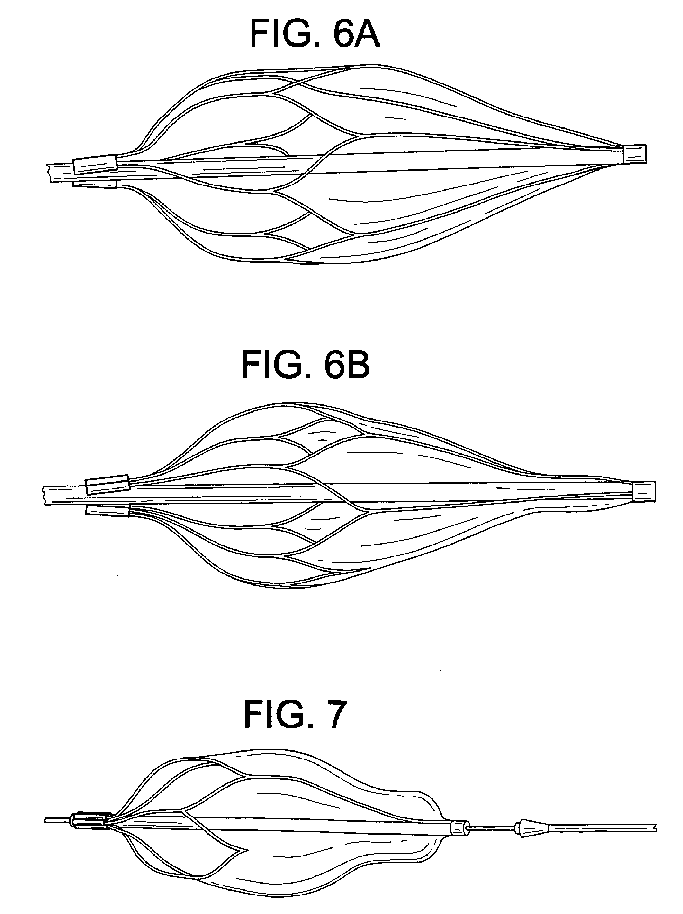

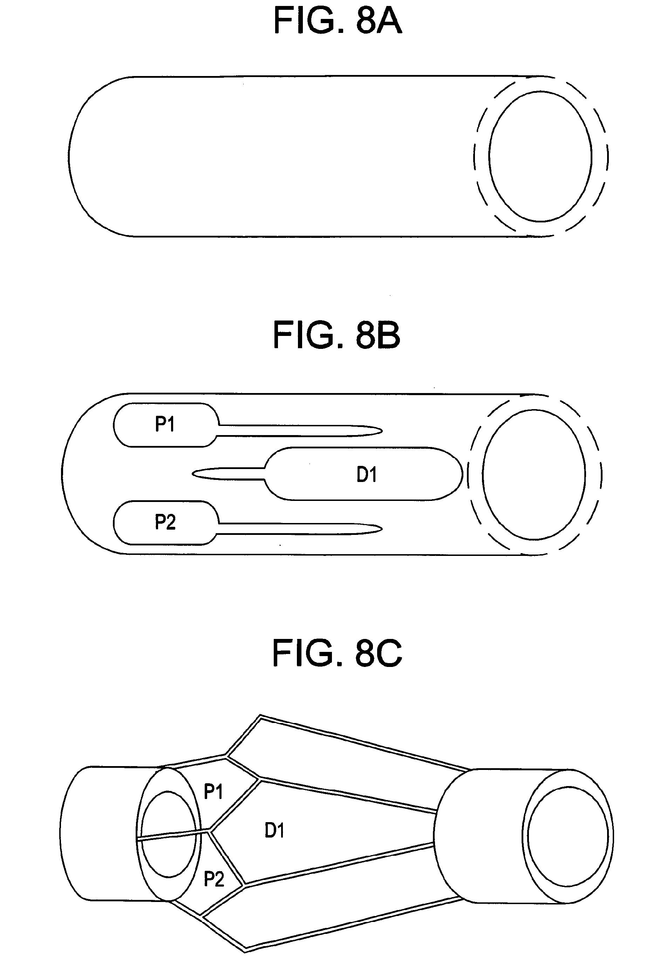

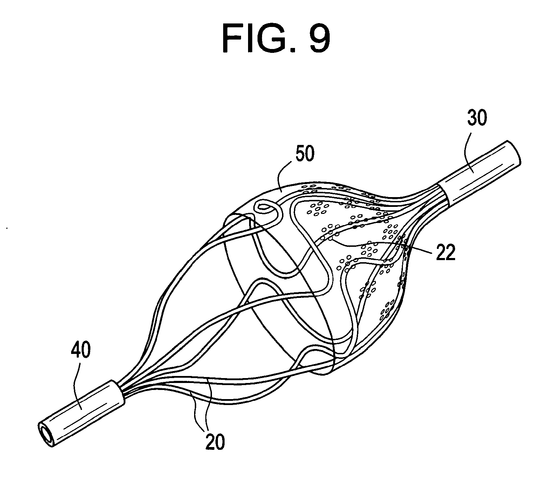

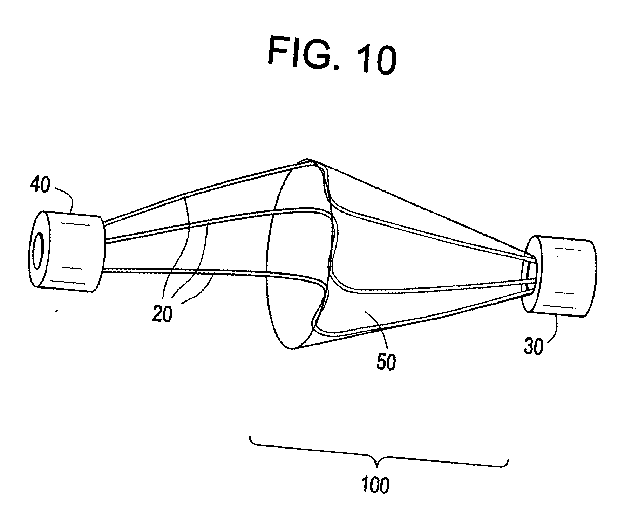

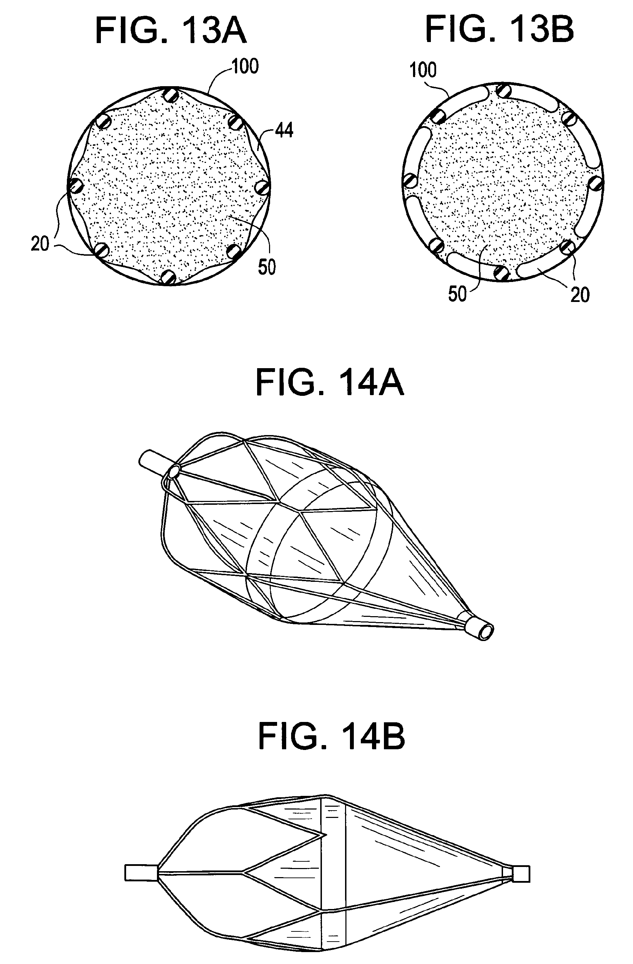

Figure 4 shows the laser cut skeleton or frame without the membrane (50) attached in accordance with the present invention. In this view the proximal support collar is shown split to allow for placement onto the mandrel. Both the frame and mandrel may then be dipped into the polymeric solution to form the filtering membrane, which attaches to the skeleton after the mandrel is removed. Figure 5A and 5B show the device without and with the membrane (50) attached. In both of these configurations the filtering membrane is substantially conical and does not incorporate the dual geometry feature as shown in figures 3A & 3B. Alternately the filtering membrane portion may be substantially spherical. Figure 6A shows the device of figure 5 with the membrane (50) attached but without the synchronous twist in the proximal portion of the skeleton. The synchronous twist when present is preferably located in the proximal portion of the supporting skeleton. Figure 6B is similar to 6A in that it too lacks the synchronous twist to the proximal portion of the frame, but unlike figure 6A, 6B shows an exemplary configuration of the dual geometry filtering configuration. This view also shows how the proximal support collar, which is cut longitudinally in half, follows guidewire (80) while still allowing for expansion away from the guide wire. In addition to allowing for mandrel insertion and removal, the split proximal support collar facilitates assembly onto guidewires (80) when required. Figure 7 shows an alternate configuration of the cut tube with the dual geometry filtering membrane (50) attached. In this exemplary view there are less distal supporting struts as compared to the exemplary configuration of figures 6A & 6B. This is a result of the cutting patterns utilized, compare figures 2A and 2B, which both utilize a similar geometry for the cutting pattern, but the pattern frequency is reduced in 2B as compared to 2A. Figure 7 shows the result when a cutting pattern with reduced frequency is utilized in three dimensions in accordance with the present invention. In each exemplary embodiment, the device including supporting skeleton members (20) and proximal and distal support rings (30 & 40) may be cut from a single tube eliminating the need for separate structural components and/or assembly of wire strands to a supporting tube. The supporting skeleton may integrally connect the proximal supporting tube with the distal supporting tube, as each of the items are cut from the same tube. As an example, laser cutting techniques for stent manufacturing can be employed to fabricate the embodiments described in accordance with the present invention. In an alternate embodiment, the skeleton frame and proximal supporting tube may be cut from a single tube and may be attached only to the filtering membrane preferably at the proximal opening of the filtering membrane eliminating the need for the distal supporting tube. This can be accomplished by utilizing a cutting pattern in which the supporting struts or skeleton may be looped back to the proximal support collar all being cut from a single tube eliminating the need for a distal support collar. Alternately, one can create a pattern in which the supporting struts or skeleton extend proximally away from the distal support collar and either loop back to the distal collar or terminate at a distance away from the distal support collar. Cutting all or most of the structural components from a single tube by laser cutting or other appropriate methods provides significant cost savings as a result of the reduced number of manufacturing process steps. Supporting struts (20) can be fabricated from tubes of differing biocompatible materials including metals, ceramics, and polymers. Preferable materials for the tubes are shape memory metals and super-elastic alloys such as nitinol. The three images shown in Figures 8A through 8C show the process steps of providing a tube, laser cutting a specific design on the tube and then expanding and tuning said cut tube into the desired shape. Expanding and tuning of the device may be accomplished with the use of mandrels and the application of heat treatments to achieve the desired configuration. In the sequence of images, on can see the two proximal openings designated P 1 & P2 as they appear in the cut tube as well as the distal opening D1 and the resulting shapes in the expanded configuration in the figure 8C. By altering the laser cut shapes and infinite set of configurations can be designed in accordance with the present invention. The struts may be cut into many shapes including those which run both substantially longitudinally aligned with the longitudinal axis of the tube as well as circumferentially resulting in the strut being substantially perpendicular with the longitudinal axis of the tube and allowing for increased circumferential support of the membrane (50) as shown in Figure 9 in accordance with the present invention. Laser drilled holes (22) can be grouped as shown in figure 9 or uniformly distributed over the entire filtering membrane (50). Figure 10 shows a similar partial side view in which approximately midway between the proximal (40) and distal (30) supporting collars or in the middle third of the device, the struts (20) are directed circumferentially for a portion of the circumference. This circumferential portion, which in this case is shown in the intermediate region of the strut, allows for improved apposition of the filtering membrane to the internal circumference of the vessel wall (100) as shown in figure 13A & 13B. In this exemplary embodiment, the filtering portion (100) is of a singular geometry and as shown is substantially conical but may be spherical or cylindrical with a rounded spherical end.

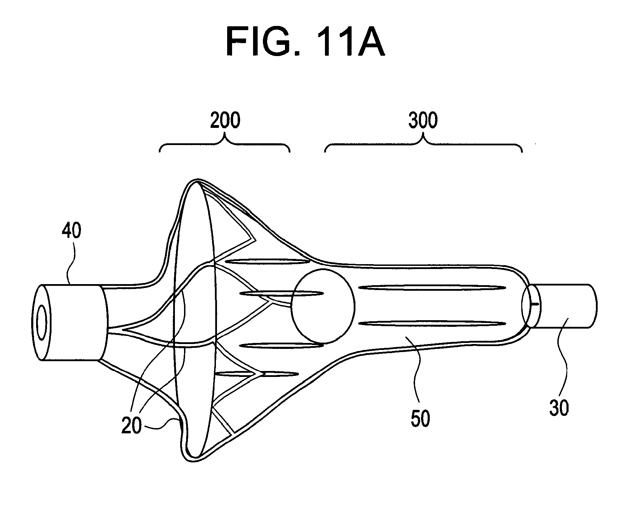

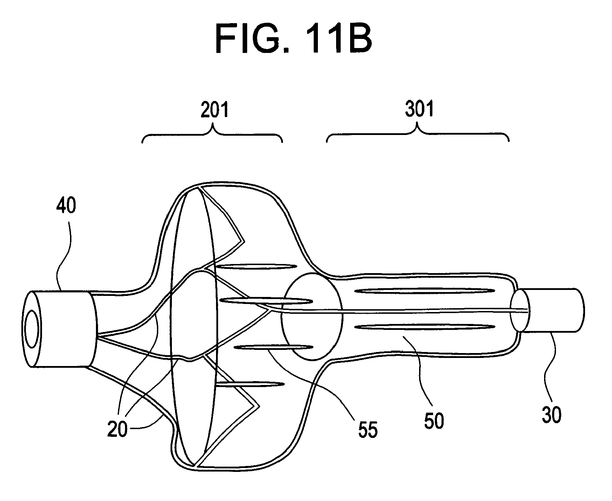

Figure 11A shows a side view of the dual geometry configuration in which the proximal (40) and distal (30) support tubes along with the intervening supporting skeleton frame (20) are all cut from the same tube, however in this exemplary embodiment, the frame is cut and tuned to a dual geometry which results in a frame which supports a substantially conical filtering portion (200) which is proximal to the substantially cylindrical filtering portion (300) also supported by the frame. Figure 11A also shows the exemplary embodiment in which laser cut slits (55) are strategically positioned on the membrane. Although these slits may be cut in any pattern, it is preferable to orientate them in a substantially longitudinal direction, which allows the device to accommodate both oversized and undersized vessel conditions. Figure 11B also shows a side view of the dual geometry configuration in which the proximal (40) and distal (30) support tubes along with the intervening supporting skeleton frame (20) are all cut from the same tube, however in this exemplary embodiment, the frame is cut and tuned to a dual geometry which results in a frame which supports a substantially spherical filtering portion (201) which is proximal to the substantially cylindrical filtering portion (301) also supported by the frame.

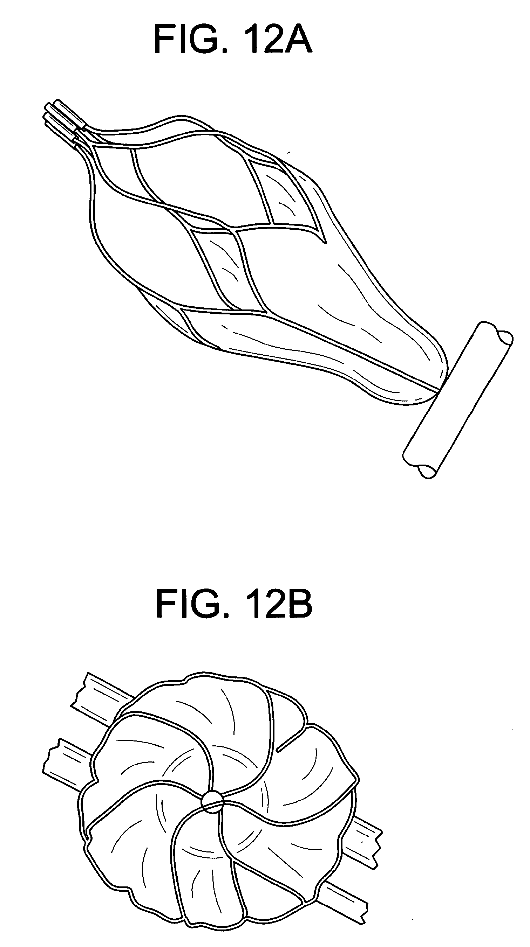

In figures 12A and 12B the dual geometry filter configuration is shown with the synchronous twist configuration imparted to the proximal portion of the supporting skeleton accomplished by tuning or programming the material to the desired shape. As stated previously the synchronous twist allows one to effectively reduce the overall length of the device which is advantageous in situations where the non-diseased operative vessel length distal to the lesion is reduced or compromised in some way.

In figures 13A and 13B, the presence of in-folding between adjacent struts is apparent resulting in a gap (44) between the filter membrane (50) and the vessel wall (100) that can allow for passage of emboli. Figure 13B shows a similar cross-section of the vessel wall (100) with a filter membrane supported by laser cut struts (20) that for a portion of the circumference are directed circumferentially. As figure 13B shows, the gap due to in-folding is eliminated due to the additional support provided by the circumferential portion of the strut (20) to the membrane against the vessel wall.

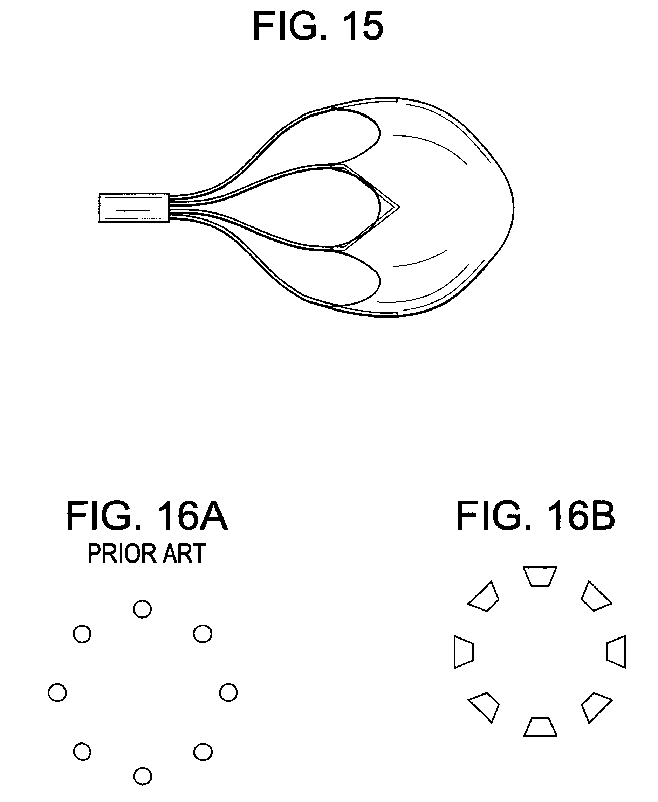

Figure 14A shows a three dimensional view of the device with a filtering membrane of a singular geometry attached without the synchronous twist having both proximal and distal support tubes and the intervening supporting skeleton frame all fabricated from a single tube before attaching the filtering membrane. Figure 14B shows a side view of the same device. Figure 15 shows a side view of the device with a proximal support collar and supporting skeleton attached to a substantially hemispherical filtering membrane in accordance with the present invention.

In figures 16A (prior art) and 16B a cross-sectional view through a representative mid section of the skeleton frame shows the improvement is available support area between the prior art assembled wire strand type device versus the laser cut tub approach. The trapezoidal cross-section of the strut cut from a tube provides additional support as compared to the circular cross-section of the wire strand due to the increase in area that would normally be in contact with the filtering membrane.

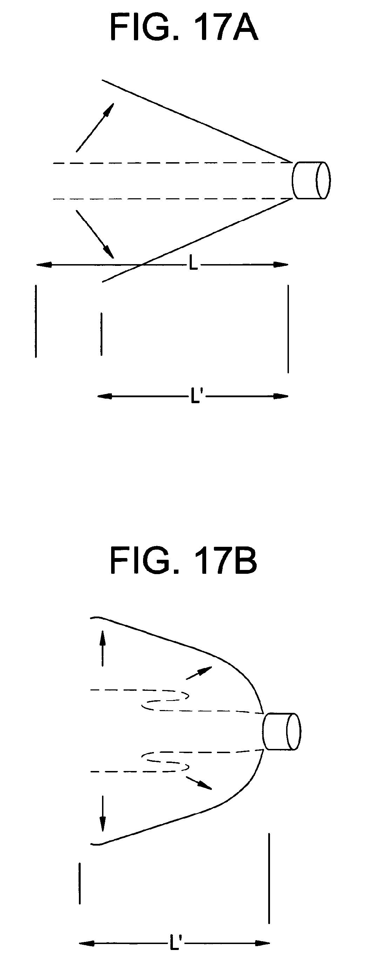

Figure 17A and figure 17B show the effect of incorporating an "s-shaped" bend in the strut member in accordance with the present invention (Figure 17B). In figure 17A, where the "s-shaped" bend is not present, one can see the effect of expansion upon the overall length, in this instance the compressed length (L) is actually longer than the expanded length (L') as measured from the distal collar. In figure 17B, one can maximize the volume of the capture space without increasing the compressed length (L') by incorporating an "s-shaped" bend in one or more of the struts comprising the skeleton frame.

## Claims

1. A vascular filtering device comprising:
a distal supporting collar having a concentric through hole adapted to slidably engage with a guide wire;
at least one primary supporting strut having a proximal end and a distal end wherein the distal end of said supporting strut is integrally attached to said distal supporting collar and the proximal end of said supporting strut is free to expand away from a guide wire; and
a compliant filtering membrane having a distal portion, wherein said membrane is operatively attached to said supporting strut and the distal portion of said membrane is operatively attached to said distal supporting collar.

2. A vascular filtering device comprising:
a proximal supporting collar having a concentric through hole adapted to slidably engage with a guide wire;
at least one primary supporting strut having a proximal end and a distal end and an intermediate portion located between the proximal and distal ends and wherein the proximal and distal ends of said supporting strut are integrally attached to said proximal supporting collar and the intermediate portion of said supporting strut is distal to said supporting collar and free to expand away from a guide wire; and
a compliant filtering membrane having a proximal opening and a distal portion, wherein the proximal opening of said membrane is operatively attached to the intermediate portion of said supporting strut.

3. A vascular filtering device comprising:
a distal supporting collar having a concentric through hole adapted to slidably engage with a guide wire;
a proximal supporting collar having a concentric through hole adapted to slidably engage with a guide wire wherein the through holes of said distal and proximal supporting collars are substantially axially aligned forming a substantially longitudinal axis;
at least one primary supporting strut having a proximal end and a distal end wherein the proximal end of said supporting strut is integrally attached to said proximal supporting collar and the distal end of said supporting strut is integrally attached to said distal supporting collar; and
a compliant substantially conical membrane having a proximal circumferential opening and a distal portion, wherein said membrane is operatively attached to both said supporting strut and to at least one of the supporting collars.

4. The filtering device of claim 3 wherein said primary supporting strut has an intermediate portion between the proximal and distal ends and the proximal and distal ends are substantially axially aligned with said substantially longitudinal axis and the intermediate portion of said primary supporting strut is substantially aligned with the circumferential opening of said substantially conical membrane.

5. The filtering device of claim 3 wherein the proximal end of said primary supporting strut is substantially axially aligned with said substantially longitudinal axis and the distal end of said primary supporting strut further comprises an S-shaped bend.

6. The filtering device of claim 3 wherein the distal end of said primary supporting strut is substantially axially aligned with said substantially longitudinal axis and the proximal end of said primary supporting strut further comprises an synchronous twist.

7. The filtering device of claim 3 wherein the distal ends of two or more said primary supporting struts are substantially axially aligned with said substantially longitudinal axis and the proximal ends of each said primary supporting strut further comprise an synchronous twist.

8. The filtering device of claim 3 wherein the distal end of a first of said at least one primary supporting strut is substantially axially aligned with said substantially longitudinal axis and the proximal end of said first primary supporting strut further comprises an synchronous twist and wherein the distal end of a second of said at least one primary supporting strut is substantially axially aligned with said substantially longitudinal axis and the proximal end of said second primary supporting strut further comprises an synchronous twist in an direction substantially opposite of the twist of the proximal end of said first primary supporting strut.

9. The filtering device of any one of claims 1 to 8 wherein said membrane is a polymeric film having at least one thru hole and the distal portion of said membrane is substantially cylindrical in shape.

10. The filtering device of any one of claims 1 to 8 wherein said membrane is a polymeric film having at least one thru hole and the distal portion of said membrane includes at least one lobe to accommodate increased loading of embolic debris.

11. The filtering device of any one of claims 1 to 8 wherein said membrane is comprises a polymeric film having a patterned set of thru holes for optimized filtering while maximizing blood flow.

12. The filtering device of any one of claims 1 to 8 wherein said membrane comprises a polymeric film having laser cut slits.

13. The filtering device of any one of claims 1 to 8 further comprising a pharmaceutically active agent.

14. The filtering device of claim 13 wherein said pharmaceutically active agent is an affinity binding compound.

15. The filtering device of claim 13 wherein said pharmaceutically active agent is an embolic lysing compound.

16. The filtering device of claim 13 wherein said pharmaceutically active agent is an antiproliferative compound.

17. The filtering device of any one of claims 1 to 8 wherein said membrane is a Nickel-Titanium Alloy thin film having at least one through hole.

18. The filtering device of claim 17 wherein said membrane incorporates an active compound.

19. The filtering device of claim 17 wherein said strut incorporates an active compound.

20. A vascular filtering device comprising:
a distal supporting collar having a concentric through hole adapted to slidably engage with a guide wire; and
a compliant Nickel-Titanium thin film filtering membrane having a proximal opening and a distal portion, wherein the distal portion of said membrane is operatively attached to said distal supporting collar.

21. The filtering device of claim 20 wherein said membrane incorporates an active compound.

22. The filtering device of any one of claims 1 to 8 wherein said supporting collar and said primary strut are a shape memory alloy.

23. The filtering device of any one of claims 1 to 8 wherein said supporting collar and said primary strut are a super-elastic alloy.

24. The filtering device of claim 22 wherein said shape-memory alloy is Nickel-Titanium Alloy.

25. The filtering device of claim 23 wherein said super-elastic alloy is Nickel-Titanium Alloy.

26. A method for fabricating a filtering device comprising the steps of:
providing a hollow tube; and
removing material from said tube such that what remains are the distal supporting collar, and at least one strut member each of which are integrally attached to each other being formed from the same tubular component.

27. The method of claim 26 wherein said tube is Nickel-Titanium Alloy.

28. The method of claim 26 wherein said removal of material is performed by laser cutting.

29. A method for fabricating a filtering device comprising the steps of:
providing a hollow tube;
removing material from said tube such that what remains are the distal supporting collar, the proximal supporting collar and at least one strut member each of which are integrally attached to each other being formed from the same tubular component; and
attaching a membrane to the distal supporting collar and at least one of the strut members.

30. The method of claim 29 further comprising the step of annealing said supporting collars and said strut after removing the material but prior to attaching the membrane in order to tune the diameter to the appropriate size and shape.

31. The method of claim 29 further comprising the step of drilling holes through said membrane to create a filtering membrane.

32. The method of claim 30 further comprising the steps of finishing by acid etching or electro-polishing or a combination of both.

33. The method of claim 30 wherein said annealing step utilizes a mandrel to support said strut during the annealing process.

34. A vascular filtering device comprising:
a distal supporting collar having a concentric through hole adapted to slidably engage with a guide wire;
a proximal supporting collar having a concentric through hole adapted to slidably engage with a guide wire;
a supporting skeleton strut having a proximal end and a distal end wherein the proximal end of said supporting skeleton is integrally attached to said proximal supporting collar and the distal end of said supporting skeleton is integrally attached to said distal supporting collar; and
a compliant filtering membrane having a distal portion, wherein said membrane is operatively attached to said supporting strut and the distal portion of said membrane is operatively attached to said distal supporting collar.

35. The vascular filtering device of claim 34 wherein said skeleton frame is configured to have a dual geometry.

36. The vascular filtering device of claim 35 wherein said dual geometry includes a substantially cylindrical filtering portion configured to capture debris while maintaining adequate blood flow through said filtering portion.

37. The vascular filtering device of claim 36 wherein said dual geometry includes a filtering portion with filtering area larger in diameter than said substantially cylindrical filtering portion configured to direct embolic debris towards said substantially cylindrical filtering portion.
